(19) Europäisches Patentamt
European Patent Office
Office européen des brevets

(11) **EP 4 681 812 A1**

(12) **EUROPÄISCHE PATENTANMELDUNG**

(43) Veröffentlichungstag:
**21.01.2026 Patentblatt 2026/04**

(21) Anmeldenummer: **25172981.0**

(22) Anmeldetag: **28.04.2025**

(51) Internationale Patentklassifikation (IPC):
**B01J 20/26** *(2006.01)* **B01J 20/10** *(2006.01)*
**B01J 20/06** *(2006.01)* **B01J 20/28** *(2006.01)*
**G01N 33/49** *(2006.01)* **G01N 33/50** *(2006.01)*
**A61B 5/15** *(2006.01)*

(52) Gemeinsame Patentklassifikation (CPC):
**A61B 5/150755; B01J 20/06; B01J 20/103; B01J 20/264; B01J 20/28047; G01N 33/491; G01N 33/5002**

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**
Benannte Erstreckungsstaaten:
**BA**
Benannte Validierungsstaaten:
**GE KH LA MA MD TN**

(30) Priorität: **29.04.2024 AT 503582024**

(71) Anmelder: **Greiner Bio-One GmbH**
**4550 Kremsmünster (AT)**

(72) Erfinder:
• **Schulz, Manuel**
**4072 Alkoven (AT)**
• **Moser, Christian**
**4690 Schwanenstadt (AT)**
• **Schwabegger, Bernhard**
**4224 Wartberg (AT)**

(74) Vertreter: **Burger, Hannes Alfred**
**Anwälte Burger & Partner**
**Rechtsanwalt GmbH**
**Rosenauerweg 16**
**4580 Windischgarsten (AT)**

(54) **TRENNGEL FÜR BLUTENTNAHMERÖHRCHEN**

(57) Die Erfindung betrifft ein Trenngel für Blutentnahmeröhrchen zur Separation von Blutserum oder -plasma von Blutzellen umfassend ein Acrylat-Copolymer, Kieselsäure und Silikonöl und/oder zumindest ein Polyalkylenglykol, wobei im Trenngel ≤ 1000 ppm, vorzugsweise ≤ 300 ppm, Lösungsmittel enthalten sind, ein Verfahren zur Herstellung des Trenngels, ein Blutentnahmeröhrchen mit dem Trenngel sowie ein Verfahren zur Herstellung des Acrylat-Copolymers, wobei zur Aufreinigung des Acrylat-Copolymers zumindest ein Lösungsmittel durch mehrstufige Destillation abgetrennt wird, sodass ein Lösungsmittelrestgehalt von ≤ 1000 ppm, insbesondere ≤ 300 ppm, erreicht wird.

**Fig.1**

EP 4 681 812 A1

**Beschreibung**

Die Erfindung betrifft ein Trenngel für Blutentnahmeröhrchen zur Separation von Blutserum oder -plasma von Blutzellen umfassend ein Acrylat-Copolymer, Kieselsäure und Silikonöl und/oder zumindest ein Polyalkylenglykol, ein Verfahren zu dessen Herstellung, ein Blutentnahmeröhrchen zur Separation von Blutserum oder -plasma von Blutzellen mit einem Trenngel, ein Verfahren zur Trennung von Blutserum oder -plasma mit einem Blutentnahmeröhrchen, sowie ein Verfahren zur Herstellung des Acrylat-Copolymers für ein Trenngel für Blutentnahmeröhrchen.

Klinische Analysetechnologien zum Nachweis biochemischen Substanzen erfordern die Trennung von Vollblut in seine Bestandteile, d. h. Serum bzw. Plasma und Blutzellen. Der abgetrennte Anteil soll möglichst frei von Blutzellen sein, um die klinischen Analysen nicht zu beeinträchtigen.

Für Laboranalysen werden zur Serum- bzw. Plasmagewinnung vielfach Blutentnahmeröhrchen mit Trenngel verwendet. Das Trenngel am Boden des Röhrchens hat eine geringere Dichte als die bei der Gerinnung aggregierten Gerinnungsproteine und Blutzellen und schiebt sich während der Zentrifugation zwischen die Blutzellen und Serum, da seine physikalische Dichte zwischen beiden Fraktionen liegt. So entsteht eine Trennschicht, die eine Kontamination der diagnostischen Probe, insbesondere des Serums mit Blutzellbestandteilen, und beispielsweise auch den Abbau von Glukose durch Blutzellen verhindert.

Für die Bestimmung von klinischen Parametern, wie z.B. Glukose, Kalium und Phosphor, muss das Serum rasch von den Blutzellen getrennt werden, da sonst die Messwerte verfälscht werden.

Durch die durch das Trenngel gebildete Diffusionsbarriere ist auch nach längerer gekühlter Lagerung die Bestimmung von Analyten der klinischen Chemie, wie z.B. Steroide, Hormone, Vitamine, Medikamente, noch möglich.

Die US5438000 beschreibt ein Serumtrennmittel mit einem ausgezeichneten Gleichgewicht der Fließ- und spezifischen Gewichtseigenschaften und einer ausgezeichneten Lagerstabilität. Das Serumtrennmittel weist ein spezifisches Gewicht bei 20°C von 1,035 bis 1,065, eine Viskosität von 100 bis 400 Pa·s und eine Fließspannung von 100 bis 400 dyn/cm$^2$ auf und umfasst (A) 100 Gewichtsteile eines Polymers mit einem spezifischen Gewicht bei 20 °C von 0,94 bis 1,06 und einer Viskosität von 10 bis 140 Pa·s, abgeleitet von einem Alkylacrylat- oder Alkylmethacrylat-Monomer; (B) 0,5 bis 10 Gewichtsteile mindestens einer Komponente, ausgewählt aus der Gruppe bestehend aus Siliciumdioxid und Bentonit; und (C) 0,01 bis 2 Gewichtsteile mindestens eines Tensids, ausgewählt aus der Gruppe bestehend aus: (C-1) Tensiden auf Fluorkohlenstoffbasis;(C-2) Tensiden auf Polyester-modifizierter Alkylpolysiloxanbasis; und (C-3) Polyether-modifizierte Alkylpolysiloxan-basierte Tenside und optional, (D) 0,01 bis 1 Gewichtsteile mindestens einer Komponente, ausgewählt aus der Gruppe bestehend aus Titandioxid und Calciumcarbonat; und (E) 0,02 bis 1 Gewichtsteile eines Haftvermittlers auf Titanbasis basierend auf 100 Gewichtsteilen des Polymers (A).

Aus der EP3734273A1 ist eine Zusammensetzung für ein Trenngel zur Trennung von Blutserum oder Blutplasma in einem Blutsammelbehälter, bekannt. Die Zusammensetzung umfasst ein (Meth)acrylsäureester-basiertes Polymer, Siliciumdioxid und ein Silikonöl, wobei das Polymer bei Raumtemperatur fließfähig ist und ein Molekulargewicht von 15.000 oder mehr und 100.000 oder weniger aufweist.

Aus dem Stand der Technik bekannte Blutentnahmeröhrchen mit Trenngel weisen einen Restlösungsmittelgehalt von Toluol oder N-Methyl-2-pyrrolidon (NMP) von größer 1000 ppm auf. Diese Stoffe sind als Gefahrstoff gemäß der Verordnung (EG) Nr. 1272/2008 eingestuft.

Bei einem aus dem Stand der Technik bekannten Blutentnahmeröhrchen mit Trenngel ist beispielsweise Toluol als Gefahrstoff mit einem Gehalt über 0,1% deklariert.

Ein weiteres am Markt befindliches Blutentnahmeröhrchen mit Trenngel enthält N-Methyl-2-pyrrolidon (NMP), ein Gefahrstoff der als SVHC-Substanz klassifiziert ist, in einer Menge von annähernd 3000 ppm.

Solche Restlösungsmittelgehalte werden in der Regel durch Lösungsmittelabtrennung über einen konventionellen Destillationsprozess erreicht.

Aufgabe der vorliegenden Erfindung ist es, die Nachteile des Standes der Technik zu überwinden und ein Mittel und ein Verfahren zur Verfügung zu stellen, mittels derer ein Benutzer in der Lage ist, eine einfache Trennung von Blutserum oder -plasma von Blutzellen vorzunehmen.

Diese Aufgabe wird durch ein Trenngel, ein Acrylat-Copolymer, ein Blutentnahmeröhrchen und einem Verfahren zur Herstellung des Trenngels und des Acrylat-Copolymers gemäß den Ansprüchen gelöst.

Das Trenngel für Blutentnahmeröhrchen zur Separation von Blutserum oder Blutplasma von Blutzellen umfassend zumindest ein Acrylat-Copolymer, Kieselsäure und Silikonöl und/oder zumindest ein Polyalkylenglykol weist ≤ 1000 ppm, vorzugsweise ≤ 300 ppm, Lösungsmittel auf, wodurch das Trenngel nicht als Gefahrstoff klassifiziert ist und dadurch die Sicherheitsvorkehrungen und Sicherheitsvorschriften für das Produkt, insbesondere für Blutentnahmeröhrchen, wo das erfindungsgemäße Trenngel eingesetzt wird, reduziert werden können.

Zudem erfolgt durch die Verwendung des erfindungsgemäßen Trenngels in einem Blutentnahmeröhrchen eine hohe Reinheit der Komponenten, welche die Gefahr der Verunreinigung der Blutprobe minimiert.

Daraus folgt auch eine bessere Probenqualität, die sich auf die nachfolgende Anwendung in der Analytik auswirken kann.

Das erfindungsgemäße Trenngel und das Verfahren zu dessen Herstellung sind sehr nachhaltig und sicher.

Es ist vorgesehen zur Polymerisation des Acrylat-Copolymers zur Herstellung einer Polymerkomponente zumindest zwei Monomere, insbesondere n-Butylacrylat und 2-Ethylhexylacrylat, ein Lösungsmittel und einen Initiator einzusetzen, womit durch die Auswahl der Komponenten, insbesondere der Monomere, eine wirtschaftliche Herstellung des Trenngels ermöglicht wird.

Vorzugsweise beträgt das Monomerverhältnis n-Butylacrylat zu 2-Ethylhexylacrylat zur Herstellung der Polymerkomponente 2:1 bis 9:1, insbesondere 3:1 bis 5:1, vorzugsweise 4:1, weil dadurch die geforderte Dichte des Polymers optimal eingestellt werden kann.

Vorzugsweise hat das Acrylat-Copolymer des Trenngels eine Dichte von 1,010 g/cm$^3$ bis 1,040 g/cm$^3$, vorzugsweise 1,025 g/cm$^3$ bis 1,035 g/cm$^3$, insbesondere zwischen 1,030 g/cm$^3$ bis 1,033 g/cm$^3$, bei 20°C, woraus ein Trenngel gebildet werden kann, das eine stabile Trennschicht mit ausreichender Festigkeit selbst bei Temperaturschwankungen bildet.

Das Acrylat-Copolymer des Trenngels weist eine Viskosität von 60 Pa·s bis 180 Pa·s, vorzugsweise 70 Pa·s bis 130 Pa·s, insbesondere 90 Pa·s bis 110 Pa·s, bei 20°C auf, wodurch eine zufriedenstellende Fließfähigkeit des Trenngels bei Raumtemperatur erreicht werden kann.

In einer bevorzugten Ausführungsform sind im Acrylat-Copolymer ≤ 1000 ppm, vorzugsweise ≤ 300 ppm, Lösungsmittel enthalten, wodurch sich ein verringerter chemischer Einfluss durch den Restgehalt des Lösungsmittels auf die Blutanalyse im Vergleich zu auf den Markt befindlichen Blutsammelröhrchen ergibt. Somit steigt auch die Probenqualität und die Analytenstabilität und besser reproduzierbare Analyseergebnisse können ebenfalls erreicht werden.

Das Acrylat-Copolymer weist vorzugsweise einen Restgehalt von ≤ 50 ppm, insbesondere ≤ 20 ppm, n-Butylacrylatmonomere und/oder von ≤ 200 ppm, vorzugsweise ≤ 100 ppm, insbesondere ≤ 80 ppm, 2-Ethylhexylacrylatmonomere auf, womit die Risiken einer Probenkontamination minimiert werden können, und die Qualität der Blutprobe und der daraus durchgeführten Analytik der klinischen Parameter verbessert wird.

Durch die geringere Menge an Monomere werden auch die Verarbeitungsprobleme beim Sterilisieren geringer.

Um eine bakterielle Infektion des Patienten und der Probe durch das Blutentnahmeröhrchen zu verhindern, wird das Blutentnahmeröhrchen während des Herstellungsprozesses durch Elektronenbestrahlung, Gammastrahlen oder Röntgenstrahlen sterilisiert, um den ISO-Standards zu entsprechen. Durch die hohe Reinheit erhöht sich die Robustheit des Trenngels im nachfolgenden Sterilisationsprozesses, da monomere Verunreinigungen zu einer ungewünschten Vernetzung des Trenngels führen würden. Diese Vernetzung könnte zu einer ungewünschten Veränderung der thixotropen Eigenschaften führen.

Silikonöl und/oder zumindest ein Polyalkylenglykol ist in einer Gesamtmenge von 0,01 Gew.-% bis 1 Gew.-%, vorzugsweise 0,05 Gew.-% bis 0,75 Gew.-%, insbesondere 0,1 Gew.-% bis 0,5 Gew.-%, im Trenngel enthalten, wodurch die erforderliche Thixotropie des Trenngels und zugleich eine gute Dispergierbarkeit der Kieselsäure erreicht wird. Auch das Auftreten einer Phasenauflösung, wo abgetrennte Komponenten niedriger Viskosität während der Lagerung aus der Phase ausfließen, kann verhindert werden.

Als Kieselsäure ist vorzugsweise pyrogene unbehandelte hydrophile und/oder modifizierte hydrophobe Kieselsäure enthalten, insbesondere in einer Menge von 0,5 Gew-% bis 5 Gew-%, vorzugsweise 1 Gew.-% bis 4 Gew.-%, insbesondere 2 Gew.-% bis 3 Gew.-%.

In einer Weiterbildung des Trenngels ist Titandioxid, insbesondere in einer Menge von 0,001 Gew.-% bis 0,1 Gew,-%, vorzugsweise 0,005 Gew.-% bis 0,08 Gew.-%, insbesondere 0,01 Gew.-% bis 0,05 Gew.-%, enthalten, wodurch zusammen mit der Kieselsäure die Dichte des Trenngels eingestellt werden kann.

Vorteilhafterweise hat das Trenngel eine Dichte von 1,038 g/cm$^3$ bis 1,058 g/cm$^3$, vorzugsweise 1,040 g/cm$^3$ bis 1,050 g/cm$^3$, insbesondere 1,044 g/cm$^3$ bis 1,048 g/cm$^3$, bei 20°C, wodurch eine stabile Trennschicht mit ausreichender Festigkeit gebildet werden kann.

Das Trenngel weist eine Viskosität von 200 Pa·s bis 520 Pa·s, vorzugsweise von 220 Pa·s bis 280 Pa·s, bei 20°C auf, wodurch die Ausbildung einer stabilen und festen Trennschicht zur Lagerung der in Phasen getrennten Blutprobe möglich ist.

Von Vorteil ist, dass das Trenngel einen thixotropen Index zwischen 1,2 und 2,2, insbesondere zwischen 1,2 und 1,7, vorzugsweise zwischen 1,3 und 1,6, aufweist, weil dadurch die Scherverflüssigung des Trenngels sichergestellt wird und die Ausbildung einer Geltrennschicht bei entsprechenden Zentrifugationsbedingungen stattfindet.

Es ist vorgesehen beim Verfahren zur Herstellung des erfindungsgemäßen Trenngels für Blutentnahmeröhrchen zur Separation von Blutserum oder -plasma umfassend zumindest ein Acrylat-Copolymer, Kieselsäure und Silikonöl und/oder zumindest ein Polyalkylenglykol, das Acrylat-Copolymer, Kieselsäure und Silikonöl und/oder zumindest ein Polyalkylenglykol und gegebenenfalls Titandioxid zu vermischen und so einen nachhaltigen und sicheren Herstellungsprozess zur Verfügung zu stellen.

Vorteilhaft erweist sich, dass zur Herstellung der Polymerkomponente des Acrylat-copolymers zumindest n-Butylacrylat in einer Menge von 30 Gew.-% bis 50 Gew.-%, vorzugsweise 35 Gew.-% bis 45 Gew.-%, insbesondere 38 Gew.-% bis 42 Gew.-%, und 2-Ethylhexylacrylat in einer Menge von 2 Gew.-% bis 20 Gew.-%, vorzugsweise 5 Gew.-% bis 15 Gew.-%, insbesondere 8 Gew.-% bis 12 Gew.-% verwendet werden, wodurch die Dichte des Polymers eingestellt werden kann.

In einer bevorzugten Ausführungsform wird als Lösungsmittel ein aromatisches Lösungsmittel, insbesondere Toluol oder Xylol zur Herstellung der Polymerkomponente, vorzugsweise in einer Menge von 30 Gew.-% bis 70 Gew.-%, vorzugsweise 40 Gew.-% bis 60 Gew.-%, insbesondere 45 Gew.-% bis 55 Gew.-%, verwendet, die eine geringere Giftigkeit im Vergleich zum häufig als Lösungsmittel verwendeten Benzol aufweisen.

Als Initiator zur Polymerisation der zumindest zwei Monomere wird ein organisches Peroxid, insbesondere 1,1,3,3-Tetramethylbutylperoxy-2-ethylhexanoat zur Herstellung der Polymerkomponente, vorzugsweise in einer Menge von 0,05 Gew.-% bis 5 Gew.-%, vorzugsweise 0,1 Gew.-% bis 1 Gew.-%, insbesondere 0,3 Gew.-% bis 0,6 Gew.-%, verwendet, wobei durch die Konzentration des Initiators die Viskosität des Polymers eingestellt werden kann. Dieser Initiator weist gegenüber dem häufig verwendeten Initiator Azobis(isobutyronitril) (AIBN) den Vorteil auf, dass kein giftiges Tetramethylsuccinnitril (TMSN) als Rückstand im Trenngel verbleibt.

In einer Weiterbildung des Verfahrens ist vorgesehen, die unbehandelte hydrophile Kieselsäure durch einen Trocknungsschritt vorzukonditionieren. Dadurch wird ein niedriger Feuchtigkeitsgehalt der Kieselsäure und somit eine konstante Qualität des daraus hergestellten Trenngels sichergestellt. Die thixotropen Eigenschaften, die sich durch Wechselwirkung von Kieselsäure und Silikonöl und/oder zumindest einem Polyalkylenglykol ausbilden, werden dadurch ebenfalls verbessert.

Ein Blutentnahmeröhrchen zur Separation von Blutserum oder -plasma von Blutzellen mit einem Trenngel zumindest aus Acrylat-Copolymer, Kieselsäure und Silikonöl und/oder zumindest einem Polyalkylenglykol, wobei im Trenngel ≤ 1000 ppm, vorzugsweise ≤ 300 ppm, Lösungsmittel enthalten sind, hat den Vorteil, dass es nicht als Gefahrstoff klassifiziert ist und somit keine Einschränkungen beim Arbeitsschutz erforderlich sind.

Durch ein Blutentnahmeröhrchen mit einem Trenngel mit einem geringen Lösungsmittelrestgehalt wird auch die Stabilität der Analyten für den Zeitraum zwischen Zentrifugation und Analyse deutlich erhöht. Dieser Stabilitätsgewinn durch die Gelbarriere ist auch ein Vorteil beim Transport und bei der Lagerung von Blut in Blutentnahmeröhrchen. Zudem ermöglicht diese stabile Barriere zwischen Serum und Blutkuchen auch eine bessere Analytenstabilität.

Durch ein erfindungsgemäßes Blutentnahmeröhrchen kann auch der Workflow von der Blutabnahme bis zur Analyse optimiert werden. So sind kurze Zentrifugationszeiten möglich, die Probenbearbeitung und Archivierung kann im Primärröhrchen erfolgen und es besteht keine Verwechslungsgefahr durch Verwendung von Sekundärröhrchen.

Vorzugsweise umfasst das erfindungsgemäße Acrylat-Copolymer für ein Trenngel für Blutentnahmeröhrchen zumindest zwei Monomere und ein Lösungsmittel, wobei ≤ 50 ppm, insbesondere ≤ 20 ppm eines ersten Monomers, und ≤ 200 ppm, vorzugsweise ≤ 100 ppm, insbesondere ≤ 80 ppm eines zweiten Monomers, sowie ≤ 1000 ppm, insbesondere ≤ 300 ppm, Lösungsmittel enthalten sind, wobei durch den niedrigen Gehalt der Restmonomere und des Lösungsmittels und somit die hohe Reinheit des Acrylat-Copolymers für ein Trenngel die Risiken einer Probenkontamination mit Monomeren oder Lösungsmittelresten minimiert werden und die Qualität der Blutprobe und der damit durchgeführten Analytik von klinischen Parametern verbessert wird.

Durch das erfindungsgemäße Verfahren zur Herstellung eines Acrylat-Copolymers für ein Trenngel für Blutentnahmeröhrchen durch radikalische Lösungsmittelpolymerisation im Zulaufverfahren aus einer Polymerlösung, wobei zur Aufreinigung des Acrylat-Copolymers zumindest das Lösungsmittel durch mehrstufige Destillation abgetrennt wird, wird ein Lösungsmittelrestgehalt von ≤ 1000 ppm, insbesondere ≤ 300 ppm, erreicht. Dadurch wird die Inverkehrbringung von Gefahrstoffen reduziert, da diese im Prozess abgetrennt und einer Wiederverwertung zugeführt werden.

Mittels des erfindungsgemäßen Verfahrens werden durch die mehrstufige Destillation auch Restmonomere aus der Polymerlösung des Acrylat-Copolymers abgetrennt, sodass ≤ 50 ppm, insbesondere ≤ 20 ppm, eines ersten Monomers, insbesondere n-Butylacrylat, und ≤ 200 ppm, vorzugsweise ≤ 100 ppm, insbesondere ≤ 80 ppm, eines zweiten Monomers, insbesondere 2-Ethylhexylacrylat, erreicht werden, wodurch ein hochreines Trenngel hergestellt werden kann, das eine reproduzierbare Analyse von Blutproben, insbesondere Analyten aus dem Blutserum oder -plasma ermöglicht.

Vorzugsweise werden die Destillationen mit Dünnschichtrotationsverdampfern kontinuierlich durchgeführt, wodurch niedrigere Werte von Lösungsmittel im Acrylat-Copolymer zur Herstellung eines Trenngels erreicht werden können.

In einer Weiterbildung des Verfahrens wird das aus dem Dünnschichtrotationsverdampfer ausgetragene Polymer einer Entspannungsverdampfungsstufe, und gegebenenfalls zuvor einer Zwischenerhitzung, zugeführt, wodurch eine bessere Entfernung des noch verbliebenen Lösungsmittels und der Restmonomere erreicht werden kann.

Vorzugsweise wird die Abtrennung des Lösungsmittels und gegebenenfalls der Restmonomere unter Vakuum durchgeführt, weil dadurch bessere Ergebnisse erzielt werden.

In einer anschließenden Kurzwegverdampfungsstufe werden das Lösungsmittel und gegebenenfalls die Restmonomere auf den gewünschten Endgehalt abdestilliert und so ein für die Weiterverarbeitung optimiertes Acrylat-Copolymer zur Verfügung gestellt.

In einer Weiterbildung des Verfahrens werden das Lösungsmittel und gegebenenfalls die Restmonomere im nachgeschalteten Kühlfallensystem ausgefroren und anschließend ausgetragen, wodurch sie einer Wiederverwertung zugeführt werden können.

Zum besseren Verständnis der Erfindung wird diese anhand der nachfolgenden Figuren näher erläutert.

Es zeigen jeweils in stark vereinfachter, schematischer Darstellung:

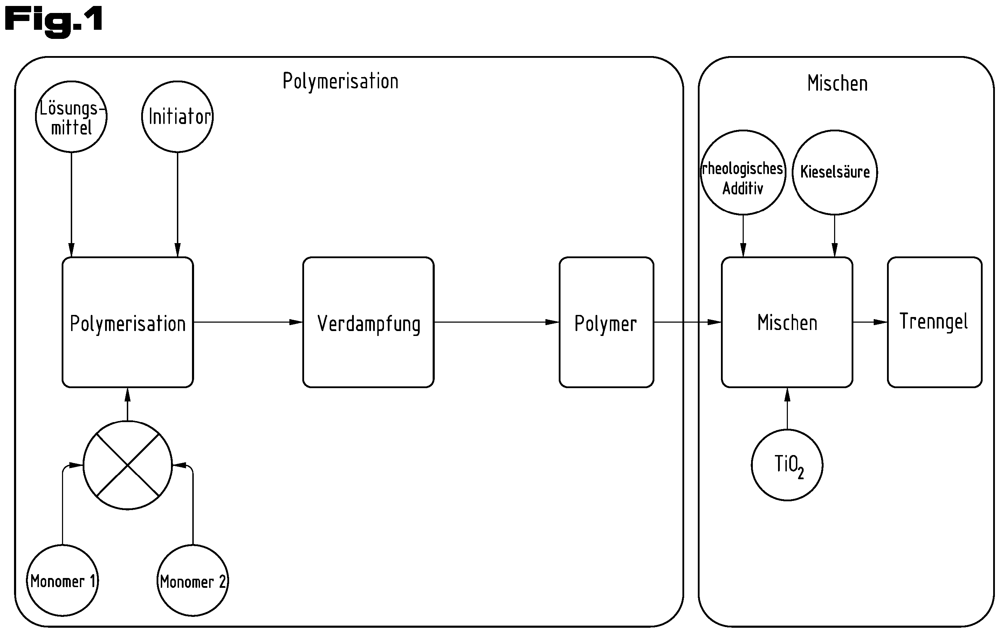

Fig. 1 ein Flussdiagramm zur Herstellung und Aufreinigung des Acrylat-Copolymers sowie zur Herstellung des Trenngels.

Die Erfindung umfasst ein Trenngel auf Basis eines hoch aufgereinigten Acrylat-Copolymers, das durch Zugabe eines rheologischen Additivs, insbesondere eines modifizierten Silikonöls und/oder zumindest eines Polyalkylenglykols, und Kieselsäure die erforderlichen rheologischen Eigenschaften in Bezug auf die Scherverflüssigung zeigt und gleichzeitig auch die ideale Dichte aufweist, um eine Trennung von Blutserum bzw. -plasma von Blutzellen zu erreichen.

Bei der Zentrifugation einer Blutprobe wirken Scherkräfte auf das Trenngel, die zu einer Verflüssigung führen. Zusätzlich wirken Auftriebskräfte während der Zentrifugation auf das Trenngel, welches sich vom Blutentnahmeröhrchenboden löst und aufsteigt. Entsprechend seiner Dichte sammelt sich das Trenngel im Bereich zwischen Blutzellen und dem Überstand, insbesondere Blutserum bzw. -plasma, und bildet dort ein stabile Trennschicht zwischen den Phasen.

Die Einstellung der korrekten Dichte und die Einhaltung enger Spezifikationsgrenzen ist neben den thixotropen Eigenschaften des Trenngels essenziell für die korrekte Funktion bei der Zentrifugation und der Ausbildung der Trennschicht. Die hohe Reinheit des Trenngels minimiert die Risiken einer Probenkontamination und verbessert die Qualität der Blutprobe und der daraus durchgeführten Analytik von klinischen Parametern und in weiterer Folge auch die Sicherheit des Materials in der weiteren Verarbeitung und Anwendung.

Die vorliegende Erfindung umfasst ein Trenngel für Blutentnahmeröhrchen zur Separation von Blutserum oder -plasma von Blutzellen umfassend ein Acrylat-Copolymer, Kieselsäure und Silikonöl und/oder zumindest ein Polyalkylenglykol, wobei im Trenngel $\leq$ 1000 ppm, vorzugsweise $\leq$ 300 ppm, Lösungsmittel enthalten sind.

Das erfindungsgemäße Trenngel wird zur Trennung von Blutserum oder Blutplasma von den übrigen Bestandteilen des Bluts verwendet und bildet eine dichte Trennschicht und Diffusionsbarriere, sodass selbst nach längerer Lagerung keine Zellinhaltstoffe in das Serum bzw. Plasma gelangen. Es wird dadurch der störende Einfluss durch die Zellen, z.B. Hämolyse, Glukoseabbau oder Kaliumfreisetzung, verhindert.

Bei der Hämolyse wird die Zellmembran der Erythrozyten zerstört, wobei intrazelluläre Bestandteile in das Serum bzw. Plasma gelangen. Wird Serum oder Plasma nicht von den Zellen getrennt, entweder durch ein Trenngel oder nach der Zentrifugation durch Abpipettieren in ein Sekundärgefäß, treten Inhaltsstoffe aus den Zellen in das Plasma oder Serum über. Die Zellwand wird bei diesem Prozess zwar nicht wie bei einer Hämolyse zerstört, die Auswirkungen auf die Probe sind allerdings ähnlich. Das Resultat ist zum Beispiel erhöhte LDH- und Kaliumwerte. Blutzucker wird durch Glykolyse abgebaut. Die Zellen nehmen dabei auch in vitro Glukose aus dem Serum bzw. Plasma auf. Dadurch verändert sich der Blutzuckerspiegel kontinuierlich im Zeitablauf. Wird Serum oder Plasma nicht von den Zellen getrennt, führt dieser Vorgang bereits nach zwei Stunden zu signifikanten Veränderungen.

Ein Trenngel gemäß der vorliegenden Erfindung wird nicht als Gefahrstoff kategorisiert und reduziert die arbeitsbedingten Gesundheitsgefahren und birgt somit weniger Risiko für das Personal im Gesundheitsbereich und bringt auch wesentliche Erleichterungen für den Arbeitgeber.

Die Bestandteile des erfindungsgemäßen Trenngels umfassen zumindest ein Acrylat-Copolymer, Kieselsäure sowie ein Silikonöl und/oder zumindest ein Polyalkylenglykol. Das erfindungsgemäße Trenngel kann auch aus zumindest einem Acrylat-Copolymer, Kieselsäure sowie einem Silikonöl und/oder zumindest einem Polyalkylenglykol bestehen.

Im Rahmen der Erfindung wird Siliciumdioxid und Kieselsäure gleichwertig verwendet und umfasst sowohl pyrogenes als auch gefälltes Siliciumdioxid bzw. Kieselsäure.

Das Trenngel zur Separation von Blutserum oder -plasma gemäß der vorliegenden Erfindung umfasst ein Acrylat-Copolymer, welches durch Polymerisation zumindest zweier unterschiedlicher polymerisierbarer Monomere gebildet ist.

In einer bevorzugten Ausführungsform werden zur Herstellung der Polymerkomponente des Acrylat-Copolymers zumindest zwei Monomere, insbesondere n-Butylacrylat (NBA) und 2-Ethylhexylacrylat (EHA), zumindest ein Lösungsmittel und zumindest ein Initiator verwendet.

Im Rahmen der Erfindung wird unter Polymerkomponente die Polymerlösung im Herstellungsprozess zur Herstellung des Acrylat-Copolymers und unter Polymer bzw. Copolymer das aufgereinigte, von Monomeren und Lösungsmittel annähernd befreite, Polymer verstanden.

Das Acrylat-Copolymer wird vorzugsweise durch radikalische Lösungsmittelpolymerisation hergestellt, bevorzugt unter Verwendung des erfindungsgemäßen radikalischen Polymerisationsverfahrens.

Polymerisierbare Monomere, die für die Herstellung des Acrylat-Copolymers des erfindungsgemäßen Trenngels verwendbar sind, sind sogenannte Alkylacrylate oder Alkylmethacrylate. Beispiele für solche Monomere sind Methyl(meth)acrylat, Ethyl(meth)acrylat, n-Butyl(meth)acrylat, i-Butyl(meth)acrylat, 2-Ethylhexyl(meth)acrylat, Lauryl(meth)acrylat, Tridecyl(meth)acrylat und Stearyl(meth)acrylat. Unter diesen sind n-Butyl(meth)acrylat oder 2-Ethylhexyl(meth)acrylat oder Copolymere, die durch die kombinierte Verwendung dieser Monomere erhalten werden, bevorzugt, da diese Polymere eine geeignete Viskosität aufweisen, leicht zu handhaben sind und in der Lage sind, Siliciumdioxid leicht zu dispergieren, um die gewünschte Dichte bzw. Viskosität einzustellen.

Darüber hinaus kann zusätzlich jedes beliebige copolymerisierbare Monomer in Kombination damit verwendet werden. Typische Beispiele für solche copolymerisierbaren Monomere sind Ethylenglykoldiacrylat, Propylenglykoldiacrylat, Neopentylglykoldiacrylat, 1,6-Hexandioldiacrylat, Diethylenglykoldiacrylat, Triethylenglykoldiacrylat, Tetraethylenglykoldiacrylat, Ethylenglykoldimethacrylat, Propylenglykoldimethacrylat, Neopentylglykoldimethacrylat, 1,6-Hexandioldimethacrylat, Diethylenglykoldimethacrylat, 1,2,3-Propantrioldi(meth)acrylat, Divinylbenzol und dergleichen.

Weitere Beispiele für das Monomer umfassen ein radikalisch polymerisierbares Monomer, das zur radikalischen Copolymerisation mit dem (Meth)acrylsäureester-Monomer fähig ist.

Beispiele für das radikalisch polymerisierbare Monomer umfassen auch aromatische Vinylmonomere, Vinylester, Vinylether, Vinylpyrrolidon und (Meth)allylether.

Als radikalisch polymerisierbares Monomer kann nur ein radikalisch polymerisierbares Monomer verwendet werden, oder es können zwei oder mehr radikalisch polymerisierbare Monomere in Kombination verwendet werden.

Beispiele für die aromatischen Vinylmonomere umfassen Styrol, $\alpha$-Methylstyrol, p-Methylstyrol, $\alpha$-Methyl-p-methylstyrol, p-Methoxystyrol, o-Methoxystyrol, 2,4-Dimethylstyrol, Chlorstyrol und Bromstyrol.

Beispiele für Vinylester umfassen (Meth)acrylat, Maleinanhydrid, Fumarat, (Meth)acrylamid, Dialkyl(meth)acrylamid und Vinylacetat. Das radikalisch polymerisierbare Monomer ist vorzugsweise das aromatische Vinylmonomer.

Das erfindungsgemäße hoch aufgereinigte Acrylat-Copolymer wird durch Polymerisation eines Alkylacrylats und/oder Alkylmethylacrylats erhalten.

In einer bevorzugten Ausführungsform beträgt das Monomerverhältnis n-Butylacrylat zu 2-Ethylhexylacrylat 2:1 bis 9:1, insbesondere 3:1 bis 5:1, vorzugsweise 4:1, zur Herstellung der Polymerkomponente für ein Trenngel. Durch das Verhältnis der Monomere kann die Dichte des Acrylat-Copolymers eingestellt werden.

Zur Herstellung der Polymerkomponente wird zumindest n-Butylacrylat in einer Menge von 30 Gew.-% bis 50 Gew.-%, vorzugsweise 35 Gew.-% bis 45 Gew.-%, insbesondere 38 Gew.-% bis 42 Gew.-%, und 2-Ethylhexylacrylat in einer Menge von 2 Gew.-% bis 20 Gew.-%, vorzugsweise 5 Gew.-% bis 15 Gew.-%, insbesondere 8 Gew.-% bis 12 Gew.-% verwendet.

Vorzugsweise beträgt daher der Anteil an der Gesamtmasse der Polymerlösung zur Herstellung des Acrylat-Copolymers für das Trenngel annähernd 50 Gew.-% Lösungsmittel, 40 Gew.-% n-Butylacrylat und 10 Gew.-% 2-Ethylhexylacrylat und ein Initiator in einer Menge von unter 1 Gew.-%.

Als Lösungsmittel wird ein aromatisches Lösungsmittel, vorzugsweise Toluol oder Xylol, zur Herstellung der Polymerkomponente zur Herstellung des Acrylat-Copolymers für das Trenngel verwendet.

In einer bevorzugten Ausführungsform wird das zumindest eine Lösungsmittel vorzugsweise in einer Menge von 30 Gew.-% bis 70 Gew.-%, vorzugsweise 40 Gew.-% bis 60 Gew.-%, insbesondere 45 Gew.-% bis 55 Gew.-%, zur Herstellung der Polymerkomponente verwendet.

Toluol ist ein CMR-Stoff. CMR-Stoffe (cancerogen, mutagen, reprotoxic) sind Stoffe, die als karzinogen, mutagen und teratogen eingestuft werden. Selbst wenn CMR-Stoffe und -Zubereitungen nur geringe oder keine akut bemerkbaren negativen Eigenschaften besitzen, kann ein Kontakt damit über eine lange Zeit in ungesunder und gefährlicher Weise für den Organismus stattfinden, ohne dass er vom Betroffenen als gefährlich wahrgenommen wird.

Da Toluol ein CMR-Stoff ist, wird die Produktsicherheit des Trenngels durch den geringen Lösungsmittelgehalt erheblich verbessert. Das erfindungsgemäße Trenngel wird nicht als Gefahrstoff eingestuft, woraus unter anderem Vorteile beim Transport und beim Arbeitsschutz folgen.

In einer bevorzugten Ausführungsform wird Toluol als Lösungsmittel zur Herstellung der Polymerkomponente des Trenngels verwendet. In einer alternativen Ausführungsform kann auch Xylol als Lösungsmittel zur Herstellung der Polymerkomponente des Trenngels verwendet werden. Xylol wird nicht als CMR-Stoff klassifiziert und ist weder als mutagen, karzinogen noch als reproduktionstoxisch einzustufen.

Als Initiator zur Polymerisation der zumindest zwei Monomere zur Herstellung der Polymerkomponente wird ein organisches Peroxid, insbesondere 1,1,3,3-Tetramethylbutylperoxy-2-ethylhexanoat, zur Herstellung der Polymerkomponente verwendet.

Vorzugsweise wird die Polymerisation der zumindest zwei Monomere mit einem organischen Peroxid als Polymerisationsinitiator durchgeführt. Als organisches Peroxid kann beispielsweise Trigonox® 421 verwendet werden, wodurch gegenüber der Verwendung von Azobis(isobutyronitril) (AIBN) als Polymerisationsinitiator kein giftiges Tetramethylsuccinnitril (TMSN) als Abbauprodukt gebildet wird.

Die zumindest zwei Monomere können aber auch mit einem Polymerisationsinitiator auf Azobasis radikalisch polymerisiert werden.

Das Acrylat-Copolymer zur Herstellung des Trenngels weist eine Dichte von 1,010 g/cm³ bis 1,040 g/cm³, vorzugsweise 1,025 g/cm³ bis 1,035 g/cm³, insbesondere zwischen 1,030 g/cm³ bis 1,033 g/cm³, bestimmt bei einer Temperatur von 20°C auf. Die Dichte des Acrylat-Copolymers wird bei einer Temperatur von 20°C mithilfe des Biegeschwinger-Dichtemessgerät DMA4500M (Anton Paar) oder einem Gaspyknometer (Pycnomatic ATC) bestimmt.

Die Viskosität des Acrylat-Copolymer liegt in einem Bereich von 60 Pa·s bis 180 Pa·s, vorzugsweise 70 Pa·s bis 130 Pa·s, insbesondere 90 Pa·s bis 110 Pa·s, bestimmt bei einer Temperatur von 20°C. Die Viskosität wird mit einem Brookfield-Viskosimeter mit Kegel-Platte Messsystem (Messkegel CPA-52Z, Winkel 3°) bei einer Scherrate von 5/sec und einer Temperatur von 20°C gemessen.

Das Acrylat-Copolymer weist nach der Aufreinigung ≤ 1000 ppm, vorzugsweise ≤ 300 ppm, Lösungsmittel auf. Gaschromatographische Messungen unterschiedlicher Chargen von aufgereinigtem Acrylat-Copolymer zeigen, dass, sofern Toluol als Lösungsmittel während der Polymerisation verwendet wird, sogar jeweils weniger als 50 ppm Toluol, insbesondere zwischen 10 ppm und 50 ppm Toluol, enthalten sind.

Das Acrylat-Copolymer weist nach der Aufreinigung einen Restgehalt von ≤ 50 ppm, insbesondere ≤ 20 ppm, n-Butylacrylat auf. Auch hier zeigten gaschromatische Messergebnisse unterschiedlicher Chargen von aufgereinigtem Acrylat-Copolymer weniger als 5 ppm des Monomers n-Butylacrylat.

Das erfindungsgemäße Trenngel beinhaltet ein aufgereinigtes Acrylat-Copolymer mit einem Restgehalt von ≤ 200 ppm, vorzugsweise ≤ 100 ppm, insbesondere ≤ 80 ppm, 2-Ethylhexylacrylatmonomer. Gaschromatographische Messungen zeigen, dass nach der Aufreinigung des Acrylat-Copolymers weniger als 20 ppm 2-Ethylhexylacrylat Monomer enthalten sind.

Die nachfolgende Tabelle 1 zeigt die Spezifikation einer bevorzugten Ausführungsform eines erfindungsgemäßen aufgereinigten Acrylat-Copolymer, welches zur Herstellung des Trenngels verwendet wird.

Tabelle 1

| Parameter | |
|---|---|
| Viskosität bei 20°C | 90 - 110 Pa·s |
| Dichte bei 20°C | 1,030 - 1,033 g/cm$^3$ |
| Toluol | ≤ 300 ppm |
| n-Butylacrylat | ≤ 20 ppm |
| 2-Ethylhexylacrylat | ≤ 80 ppm |

Die Mengenangaben in Zusammenhang mit der Zusammensetzung des Trenngels sind im Nachfolgenden bezogen auf 100 Gew.-% der Zusammensetzung des Trenngels zur Trennung von Blutserum oder Blutplasma.

Gemäß der Erfindung ist neben dem Acrylat-Copolymer auch Silikonöl und/oder zumindest ein Polyalkylenglykol im Trenngel enthalten. Der Gesamtgehalt des Silikonöls und/oder des zumindest einen Polyalkylenglykols beträgt vorzugsweise 0,01 Gew.-% bis 1 Gew.-%, vorzugsweise 0,05 Gew.-% bis 0,75 Gew.-%, insbesondere 0,1 Gew.-% bis 0,5 Gew.-%, bezogen auf 100 Gew.-% der Zusammensetzung zur Trennung von Blutserum oder Blutplasma. Weicht der Gesamtgehalt des Silikonöls und/oder des zumindest einen Polyalkylenglykols von den oben definierten Grenzen ab, kann dies zum Auflösen der Phasentrennung führen und Phasen unterschiedlicher Dichte würden sich vermischen.

Als rheologisches Additiv ist vorzugsweise Silikonöl auf Basis von Polyether-modifiziertem Polysiloxan im Trenngel enthalten. Silikonöl dient zum Einstellen der Viskosität und es kann dadurch die erforderliche Thixotropie in Kombination mit der Kieselsäure erreicht werden.

Beispiele für mögliche Silikonöle umfassen Dimethylsilikonöle, Methylphenylsilikonöle, Methylwasserstoffsilikonöle, alkylmodifizierte Silikonöle, aralkylmodifizierte Silikonöle, fluormodifizierte Silikonöle, polyethermodifizierte Silikonöle, aminomodifizierte Silikonöle, epoxidmodifizierte Silikonöle, phenolmodifizierte Silikonöle, carboxymodifizierte Silikonöle, methacrylatmodifizierte Silikonöle und alkoxymodifizierte Silikonöle.

Als Silikonöl kann nur ein Silikonöl verwendet werden, oder es können auch zwei oder mehr Silikonöle in Kombination verwendet werden.

Als Alternative oder in Kombination mit Silikonöl kann auch zumindest ein Polyalkylenglykol mit einer zahlenmittleren Molmasse (Mn) aus einem Bereich von 100 bis 10000 Da, vorzugsweise 200 bis 5000 Da, insbesondere 400 bis 4000 Da, als rheologisches Additiv im Trenngel enthalten sein. Mögliche Polyalkylenglykole sind Polyethylenglykol (PEG) (R = $CH_2$-$CH_2$-O) wie zum Beispiel Polyethylenglykol-400, Polypropylenglykol (PPG) (R = $CH_2$-$CH_2$-($CH_3$)-O) wie zum Beispiel Polypropylenglykol-400 und Polypropylenglykol-1000, Block-Co-Polymere und statistische Co-Polymere aus Ethylenoxid und Propylenoxid Einheiten wie zum Beispiel Poly(propylenglykol)-block-poly(ethylenglykol)-block-poly(propylenglykol), Poly(ethylenglykol)-block-poly(propylenglykol)-block-poly(ethylenglykol). Weiters können Polyalkylenglykole deren OH Endgruppen durch Alkohole vollständig oder teilweise substituiert sein können, wie zum Beispiel Polypropylenglykolmonobutylether, eingesetzt werden.

Im erfindungsgemäßen Trenngel ist auch Siliciumdioxid enthalten. Siliciumdioxid ist vorzugsweise in Form von synthetischer, amorpher Kieselsäure, insbesondere in einer Menge von 0,5 Gew-% bis 5 Gew-%, vorzugsweise 1 Gew.-% bis 4 Gew.-%, insbesondere 2 Gew.-% bis 3 Gew.-% enthalten.

Als Kieselsäure kann sowohl unbehandelte hydrophile als auch modifizierte hydrophobe Kieselsäure oder eine Mischung daraus verwendet werden.

Modifizierte Kieselsäuren enthalten auf der Oberfläche kovalent gebundene organische Gruppen, um hydrophobe Eigenschaften zu erzielen. Häufig erfolgt die Herstellung durch Reaktion der frei zugänglichen Silanolgruppen (Si-OH) auf der Oberfläche der unbehandelten Kieselsäure mit Silanen, Silazanen oder Siloxanen.

Beispiele für diese organischen Verbindungen umfassen Dimethyldichlorsilan, Octamethylcyclotetrasiloxan, Polydimethylsiloxan, Methacrylsilan, Octylsilan, Hexamethyldisilazan, Hexadecylsilan.

Kieselsäure wird zur Gelnetzwerkbildung und als Mittel zur Steuerung der Dichte verwendet. Mit der Kieselsäure kann in Kombination mit dem Silikonöl und/oder zumindest einem Polyalkylenglykol sowohl die Thixotropie als auch die Dichte des Trenngels eingestellt werden.

Bevorzugt liegt der mittlere Äquivalentdurchmesser der Partikel der Kieselsäure in einem Bereich von 10 nm bis 100 nm, insbesondere 5 nm bis 30 nm. Der Partikeldurchmesser wird mittels Laserbeugungsmethode bestimmt.

Das Trenngel kann ein weiteres anorganisches Pulver, insbesondere Titandioxid, insbesondere in einer Menge von 0,001 Gew.-% bis 0,1 Gew,-%, vorzugsweise 0,005 Gew.-% bis 0,08 Gew.-%, insbesondere 0,01 Gew.-% bis 0,05 Gew.-%, enthalten.

Titandioxid und Kieselsäure sind als nichtlösliche Teilchen im Gel eingeschlossen und werden zur exakten Einstellung der Dichte des Trenngels verwendet.

Es können noch weitere anorganische Pulver wie Zinkoxidpulver, Aluminiumoxidpulver, feines Glaspulver, Talkpulver, Kaolinpulver, Bentonitpulver und Zirkoniumpulver im Trenngel enthalten sein.

Das Trenngel gemäß der vorliegenden Erfindung kann andere Komponenten als die oben beschriebenen Komponenten enthalten, solange die Wirkungen der vorliegenden Erfindung nicht beeinträchtigt werden, insbesondere die Separationseigenschaft des Trenngel noch gewährleistet ist sowie das nicht Vorliegen eines Gefahrstoffes beibehalten wird.

Als mögliche weitere Komponenten der vorliegenden Erfindung kann beispielsweise ein Antioxidans und einen Farbstoff enthalten sein. Als jede weitere Komponente kann nur eine Komponente verwendet werden, oder es können zwei oder mehr Komponenten in Kombination im Trenngel enthalten sein.

Das erfindungsmäße Trenngel weist eine Dichte von 1,038 g/cm$^3$ bis 1,058 g/cm$^3$, vorzugsweise 1,040 g/cm$^3$ bis 1,050 g/cm$^3$, insbesondere 1,044 g/cm$^3$ bis 1,048 g/cm$^3$, bei 20°C auf. Die Dichte des Trenngels im ausgewählten Bereich ermöglicht es eine stabile Trennwand zwischen Blutserum bzw. -plasma und den restlichen Bestandteilen des Bluts mit ausreichender Festigkeit selbst bei Vorliegen einer geringen Menge an Blutzellen bzw. Blutzellbestandteilen, niedrigen Temperaturen und/oder unter Anwendung einer geringen Zentrifugalkraft auszubilden.

Die Dichte des Trenngels wird mithilfe des Biegeschwinger-Dichtemessgeräts DMA4500M (Anton Paar) oder einem Gaspyknometer (Pycnomatic ATC) bei einer Temperatur von 20°C bestimmt.

Die Viskosität des Trenngel liegt in einem Bereich von 200 Pa·s bis 520 Pa·s, vorzugsweise von 220 Pa·s bis 280 Pa·s, bei einer Temperatur von 20°C. Die Viskosität wird mit einem Brookfield-Viskosimeter mit Kegel-Platte Messsystem (Messkegel CPA-52Z, Winkel 3°) bei einer Scherrate von 1/sec und einer Temperatur von 20°C gemessen, bis sich ein stabiler Messwert eingestellt hat.

Der thixotrope Index (TI) des erfindungsgemäßen Trenngels liegt zwischen 1,2 und 2,2, insbesondere zwischen 1,2 und 1,7, vorzugsweise zwischen 1,3 und 1,6.

Der thixotrope Index ist der Quotient aus zwei Viskositäts-Messwerten bei unterschiedlichen Scherraten. Dazu wird die Viskosität wird mit einem Brookfield-Viskosimeter mit Kegel-Platte Messsystems (Messkegel CPA-52Z, Winkel 3°) bei einer Temperatur von 20°C gemessen. Zuerst wird bei einer Scherrate von 1/sec , bis sich ein stabiler Messwert eingestellt hat, anschließend bei einer Scherrate von 5/sec, bis sich ein stabiler Messwert eingestellt hat, gemessen. Die Berechnung des thixotropen Index erfolgt nach Formel 1.

Formel 1

$$TI = \frac{Viskosität\ bei\ Scherrate\ 1/sec}{Viskosität\ bei\ Scherrate\ 5/sec}$$

Gaschromatographische Messungen des Trenngels zeigen einen niedrigen Gehalt des Lösungsmittels Toluol und geringe Mengen der Monomere n-Butylacrylat und 2-Ethylhexylacrylat. Die Messung erfolgt mittels Headspace-Gaschromatographie mit Flammenionisationsdetektor. Das erfindungsgemäße Trenngel weist unter Verwendung eines aufgereinigten Acrylat-Copolymers zu dessen Herstellung einen Restgehalt von ≤ 50 ppm, insbesondere ≤ 20 ppm, n-Butylacrylatmonomer und einen Restgehalt von ≤ 200 ppm, vorzugsweise ≤ 100 ppm, insbesondere ≤ 80 ppm, 2-Ethylhexylacrylatmonomer auf.

In besonders bevorzugten Ausführungsformen weist das Trenngel einen Restgehalt von ≤ 5 ppm n-Butylacrylatmonomer und von ≤ 20 ppm 2-Ethylhexylacrylatmonomer und gleichzeitig einen Toluolrestgehalt von ≤ 50 ppm auf.

Die nachfolgende Tabelle 2 zeigt die Spezifikation einer möglichen Ausführungsvariante des erfindungsgemäßen Trenngels.

Tabelle 2

| Parameter | |
|---|---|
| Viskosität bei 20°C | 200 - 520 Pa·s |
| Thixotroper Index | 1,2 - 2,2 |
| Dichte bei 20°C | 1,038 - 1,058 g/cm$^3$ |
| Toluol (GC) | ≤ 300 ppm |
| n-Butylacrylat (GC) | ≤ 20 ppm |
| 2-Ethylhexylacrylat (GC) | ≤ 80 ppm |

Die Herstellung des erfindungsgemäßen Trenngels für Blutentnahmeröhrchen zur Separation von Blutserum oder -plasma kann beispielsweise durch Mischen des erfindungsmäßen aufgereinigten Acrylat-Copolymers, Kieselsäure und Silikonöl und/oder zumindest einem Polyalkylenglykol und gegebenenfalls Titandioxid und gegebenenfalls anderen optionalen Komponenten erfolgen.

In einer bevorzugten Ausführungsform wird die unbehandelte hydrophile Kieselsäure durch einen Trocknungsschritt vorkonditioniert. Die Trocknung kann durch Temperaturerhöhung, Druckabsenkung (Vakuum) oder Durchströmen mit trockenem Gas (Schleppgas) erfolgen. Die Anwendung einer Kombination von zwei oder aller drei dieser Verfahren erhöht die Effizienz der Trocknung in Hinblick auf die Zeitdauer und erzielbarer Restfeuchtigkeit. Die Kieselsäure wird in einem beheizten Behälter bei 60 °C und unter Vakuum mit einem Schleppgas (trockene Druckluft) durchströmt. Um den Fortschritt der Trocknung zu überwachen, wird die Feuchtigkeit des Abgases gemessen und bei einem Wert < 1% relative Feuchte im Abgasstrom beendet.

Fig. 1 zeigt ein Flussdiagramm des Herstellungsverfahrens des erfindungsgemäßen Trenngels umfassend die Polymerisation und Aufreinigung des Acrylat-Copolymers und das Mischen der Komponenten des erfindungsgemäßen Trenngels.

Zur Herstellung der Polymerkomponente des Acrylat-Copolymers werden zumindest zwei Monomere, vorzugsweise die beiden Monomere n-Butylacrylat und 2-Ethylhexylacrylat, vermischt und der Initiator anschließend in das vorgelegte siedende Lösungsmittel im Zulaufverfahren dem Reaktor zur radikalischen Lösungsmittelpolymerisation zugeführt.

Um das Lösungsmittel und die Monomere nach der Polymerisation aus der Polymerkomponente des Acrylat-Copolymers abzutrennen wird eine Verdampfung in mehreren Stufen durchgeführt, wobei vorzugsweise eine Dünnschichtdestillation gefolgt von einer Entspannungsverdampfungsstufe und eine Kurzwegverdampfungsstufe durchgeführt wird.

Das Verfahren zur Herstellung des Trenngels kann unter Anwendung eines bekannten Mischers, wie beispielsweise eines Planetenmischers, einer Kugelmühle oder einer Dispergiermaschine erfolgen.

Beim Verfahren zur Herstellung eines Trenngels für Blutentnahmeröhrchen zur Separation von Blutserum oder -plasma umfassend ein Acrylat-Copolymer, Kieselsäure und Silikonöl und/oder zumindest einem Polyalkylenglykol, werden das Acrylat-Copolymer, Kieselsäure und Silikonöl und/oder zumindest einem Polyalkylenglykol und gegebenenfalls Titandioxid in einem Vakuumplanetendissolver bei 60°C, einem Unterdruck von ≤ 40 mbar und für ungefähr 60 Minuten gemischt. Vorzugsweise beträgt die Umfangsgeschwindigkeit der Dissolverscheibe ca. 18 m/s bis 30 m/s. Eine höhere Mischgeschwindigkeit erweist sich im Mischprozess von Vorteil. Dabei kann ein etabliertes Verfahren zur Herstellung des erfindungsgemäßen Trenngels verwendet werden.

Um die erforderliche Thixotropie zu erreichen und eine zufriedenstellende Dispergierbarkeit der Kieselsäure zu erzielen, werden zuerst das Acrylat-Copolymer und das Silikonöl und/oder das zumindest eine Polyalkylenglykol gemischt und anschließend die Kieselsäure und gegebenenfalls das Titandioxid eingemischt und gegebenenfalls weitere Komponenten zur resultierenden Mischung zugegeben. In einer bevorzugten Ausführungsform wird die Kieselsäure vor ihrer Zugabe zum Acrylat-Copolymer-Silikonölgemisch und/oder Acrylat-Copolymer-Polyalkylenglykolgemisch vorkonditioniert, indem sie getrocknet wird.

Das erfindungsgemäße Trenngel ist in einem Blutentnahmeröhrchen zur Separation von Blutserum oder -plasma enthalten. Gemäß der vorliegenden Erfindung umfasst ein Röhrchen zumindest das erfindungsgemäße Trenngel, wobei das Trenngel im Blutentnahmeröhrchen enthalten ist. Bevorzugt ist das Trenngel im Bereich des geschlossenen Endes des Blutentnahmeröhrchens enthalten.

Zur Gewinnung von Blutserum wird die Gerinnung des Bluts aktiviert, z.B. durch Silicapartikel an der Blutentnahmeröhrcheninnenwand oder durch zusätzliches Thrombin im Serumröhrchen.

VACUETTE® CAT Serum Gerinnungsaktivator Röhrchen sind mit mikronisierten Silicapartikeln beschichtet, die die Gerinnung aktivieren und durch Wenden des befüllten Blutabnahmeröhrchens in der Blutprobe suspendiert werden.

VACUETTE® CAT Serum Fast Separator Röhrchen enthalten ein Trenngel am Blutentnahmeröhrchenboden. Im Gegensatz zu herkömmlichen Serumröhrchen enthält das Serum Fast Röhrchen zusätzlich Thrombin, um den Gerinnungsprozess zu beschleunigen.

Serumröhrchen werden bei Bestimmungen für die klinische Chemie und Immunologie, Hormone, therapeutisches Drug Monitoring (TDM) und Serologie verwendet.

Versetzt man das Vollblut mit Antikoagulantien, gerinnt es nicht, und man erhält durch die Zentrifugation das Plasma. Das Plasma enthält alle Gerinnungs- und Fibrinolysefaktoren in aktiver Form. Plasmaröhrchen werden ebenfalls zur Bestimmung von Blutparametern der klinischen Chemie verwendet.

Das Trenngel wandert während der Zentrifugation zur Grenzfläche zwischen flüssigem und Blutzellenanteil des Blutes, wo es eine stabile Barriere bildet und den Überstand von den Zellbestandteilen trennt. Blutentnahmeröhrchen werden bei Bestimmungen für die klinische Chemie und Immunologie, Hormone, TDM und Serologie verwendet.

Das Material des Blutentnahmeröhrchens kann beispielsweise ein thermoplastischer Kunststoff wie Polyethylen (PE), Polypropylen (PP), Polystyrol (PS), Polyethylenterephthalat (PET), Polyethylene furan-2,5-dicarboxylat (PEF), Polymethylmethacrylat, Polyacrylnitril, Polyamid, Acrylnitril-Styrol-Copolymere und Ethylen-Vinylalkohol-Copolymere; oder ein duroplastischer Kunststoff, wie etwa ungesättigte Polyesterharze, Epoxidharze und Epoxid-Acrylatharze; modifizierte Naturharze, wie etwa Celluloseacetat, Cellulosepropionat, Ethylcellulose und Ethylchitin; Silikate wie Natronkalkglas, Phosphosilikatglas und Borosilikatglas, Gläser wie Quarzglas und Kombinationen davon oder Materialien, die hauptsächlich eines der oben genannten enthalten, sein.

Erfindungsgemäße Blutentnahmeröhrchen sind vorzugsweise aus PET-Kunststoff hergestellt und bieten verbesserte Sicherheit und Hygiene bei der Probenentnahme mit dem Vorteil, dass sie glasklar sind.

Das Blutentnahmeröhrchen kann mit einem Verschlusselement, vorzugsweise einem Verschlussstopfen, verschlossen sein.

Vorzugsweise ist der Innenraum des Blutentnahmeröhrchen evakuiert und weist einen Verschlussstopfen auf.

Um Infektionen zu verhindern, wird das Blutentnahmeröhrchen sterilisiert, beispielsweise durch Elektronenbestrahlung Gammastrahlen oder Röntgenstrahlen.

Zudem können an der Innenwand des Blutentnahmeröhrchen weitere Komponenten angebracht sein. So kann beispielsweise ein Blutgerinnungsbeschleuniger angebracht werden.

Wird das Blutentnahmeröhrchen zur Gewinnung von Blutplasma verwendet, ist ein Antikoagulans in Blutentnahmeröhrchen enthalten, welches ebenfalls an der Blutentnahmeröhrcheninnenwand haften kann. Es können Antikoagulanzien, wie Heparin, EDTA oder Citrat, bzw. andere aus dem Stand der Technik bekannte Stoffe angebracht sein.

In einer bevorzugten Ausführungsform wird 1,4 g Trenngel zur Trennung von Blutserum bzw. -plasma in ein mit Unterdruck versehenes 100 mm langes und einen 16 mm Außendurchmesser aufweisendes Blutentnahmeröhrchen mit Gummistopfen abgefüllt und anschließend sterilisiert.

Beim Verfahren zur Trennung von Blutserum oder -plasma mit einem Blutentnahmeröhrchen gemäß der vorliegenden Erfindung wird das Vollblut im Blutentnahmeröhrchen zentrifugiert, um Blutplasma oder Blutserum in ausreichender Menge und Qualität zu gewinnen. Das Vollblut wird zuvor im Blutentnahmeröhrchen gesammelt. Durch das Zentrifugieren gelangt das Trenngel zwischen die Schicht beinhaltend die Blutzellen und die Schicht beinhaltend das Blutserum bzw. -plasma. Die Dichte der Blutzellen ist höher als jene des Blutplasmas bzw. - serums und liegt in einem Bereich von circa 1,06 g/cm$^3$ bis 1,11 g/cm$^3$, wohingegen die Dichte des Blutserums bzw. -plasmas in einem Bereich von 1,025 g/cm$^3$ und 1,030 g/cm$^3$ liegt. Die Dichte des Trenngels liegt dazwischen und beträgt 1,038 g/cm$^3$ bis 1,058 g/cm$^3$.

Das Acrylat-Copolymer für ein Trenngel für Blutentnahmeröhrchen umfasst zumindest zwei Monomere und ein Lösungsmittel, mit ≤ 50 ppm, insbesondere ≤ 20 ppm, eines ersten Monomers, insbesondere n-Butylacrylat, und ≤ 200 ppm, vorzugsweise ≤ 100 ppm, insbesondere ≤ 80 ppm, eines zweiten Monomers, insbesondere 2-Ethylhexylacrylat sowie einem Lösungsmittelanteil, insbesondere von Toluol, von ≤ 1000 ppm, insbesondere ≤ 300 ppm.

Zur Herstellung der Polymerkomponente des Acrylat-Copolymers beträgt das Verhältnis der Monomere n-Butylacrylat zu 2-Ethylhexylacrylat 2:1 bis 9:1, insbesondere 3:1 bis 5:1, vorzugsweise 4:1.

Zur Herstellung der Polymerkomponente wird zumindest n-Butylacrylat in einer Menge von 30 Gew.-% bis 50 Gew.-%, vorzugsweise 35 Gew.-% bis 45 Gew.-%, insbesondere 38 Gew.-% bis 42 Gew.-%, und 2-Ethylhexylacrylat in einer Menge von 2 Gew.-% bis 20 Gew.-%, vorzugsweise 5 Gew.-% bis 15 Gew.-%, insbesondere 8 Gew.-% bis 12 Gew.-%, verwendet.

Als Lösungsmittel wird ein aromatisches Lösungsmittel, vorzugsweise Toluol und/oder Xylol, zur Herstellung der Polymerkomponente in einer Menge von 30 Gew.-% bis 70 Gew.-%, vorzugsweise 40 Gew.-% bis 60 Gew.-%, insbesondere 45 Gew.-% bis 55 Gew.-%, verwendet.

Ein organisches Peroxid, vorzugsweise 1,1,3,3-Tetramethylbutylperoxy-2-ethylhexanoat wird vorzugsweise in einer Menge von 0,05 Gew.-% bis 5 Gew.-%, vorzugsweise 0,1 Gew.-% bis 1 Gew.-%, insbesondere 0,3 Gew.-% bis 0,6 Gew.-%, als Initiator der Polymerisation der zumindest zwei Monomere zur Herstellung der Polymerkomponente verwendet.

Vorzugsweise werden zur Herstellung der Polymerkomponente zur Herstellung des Acrylat-Copolymers für das Trenngel annähernd 50 Gew.-% Lösungsmittel, 40 Gew.-% n-Butylacrylat und 10 Gew.-% 2-Ethylhexylacrylat und ein Initiator in einer Menge von unter 1 Gew.-% verwendet.

Das aufgereinigte Acrylat-Copolymer weist eine Dichte von 1,01 g/cm$^3$ bis 1,04 g/cm$^3$, vorzugsweise 1,025 g/cm$^3$ bis 1,035 g/cm$^3$, insbesondere zwischen 1,030 g/cm$^3$ bis 1,033 g/cm$^3$, bei 20°C auf und eine Viskosität von 60 Pa·s bis 180 Pa·s, vorzugsweise 70 Pa·s bis 130 Pa·s, insbesondere 90 Pa·s bis 110 Pa·s, bei 20°C auf.

Das erfindungsgemäße aufgereinigte Acrylat-Copolymers zur Herstellung eines Trenngels für ein Blutentnahmeröhrchen weist einen Restgehalt von ≤ 50 ppm, insbesondere ≤ 20 ppm, n-Butylacrylatmonomer und einen Restgehalt von ≤ 200 ppm, vorzugsweise ≤ 100 ppm, insbesondere ≤ 80 ppm, 2-Ethylhexylacrylatmonomer auf.

In besonders bevorzugten Ausführungsformen weist das Acrylat-Copolymer einen Restgehalt von ≤ 5 ppm n-Butylacrylatmonomer und von ≤ 20 ppm 2-Ethylhexylacrylatmonomer und gleichzeitig einen Toluolrestgehalt von ≤ 50 ppm auf.

Das erfindungsgemäße Acrylat-Copolymer für ein Trenngel für Blutentnahmeröhrchen wird vorzugsweise durch radikalische Lösungsmittelpolymerisation im Zulaufverfahren aus einer Polymerlösung hergestellt, wobei zur Aufreinigung des Acrylat-Copolymers zumindest das Lösungsmittel durch mehrstufige Destillation abgetrennt wird, sodass ein Lösungsmittelrestgehalt von ≤ 1000 ppm, insbesondere ≤ 300 ppm, erreicht wird.

Die Starttemperatur der Polymerisation für das Acrylat-Copolymers liegt beim Siedepunkt des jeweils verwendeten Lösungsmittels. Die einzelnen Monomere werden über einen längeren Zeitraum von zumindest 60 Minuten, vorzugsweise 120 Minuten zugeführt, wobei das Monomerverhältnis NBA zu EHA vorzugsweise 4:1 beträgt. Gleichzeitig werden über denselben Zeitraum oder länger, insbesondere bis zu 240 Minuten der Initiator der Polymerisationslösung zugeführt.

In einer alternativen Ausführungsform kann die Herstellung der Polymerkomponente des Acrylat-Copolymers für ein Trenngel für Blutentnahmeröhrchen auch unter Verwendung eines Polymerisationsinitiator auf Azobasis , beispielsweise Azobis(isobutyronitril) (AIBN) erfolgen.

Durch die mehrstufige Destillation werden auch Restmonomere aus der Polymerlösung des Acrylat-Copolymers abgetrennt, sodass ≤ 50 ppm, insbesondere ≤ 20 ppm, eines ersten Monomers, insbesondere n-Butylacrylat, und ≤ 200 ppm, vorzugsweise 100 ppm, insbesondere ≤ 80 ppm, eines zweiten Monomers, insbesondere 2-Ethylhexylacrylat, erreicht werden.

Die Destillation wird mit Dünnschichtrotationsverdampfern kontinuierlich durchgeführt.

Die Dünnschichtverdampfung flüchtiger Stoffe, hauptsächlich Toluol und/oder Xylol, aber auch der Monomere n-Butylacrylat und 2-Ethylhexylacrylat, erfolgt bei einer Temperatur zwischen 130°C und 150°C Manteltemperatur und einem Druck von 90 mbar bis 100 mbar.

Das aus dem Dünnschichtrotationsverdampfer ausgetragene Polymer wird einer Flashverdampfungsstufe bzw. Entspannungsverdampfungsstufe, und gegebenenfalls zuvor einer Zwischenerhitzung, zugeführt.

Die Abtrennung des Lösungsmittels und gegebenenfalls der Restmonomere wird unter Vakuum durchgeführt.

Gegebenenfalls wird in einer anschließenden Kurzwegverdampfungsstufe das Lösungsmittel und gegebenenfalls die Restmonomere auf den gewünschten Endgehalt abdestilliert.

Die Kurzwegverdampfung erfolgt vorzugsweise bei einer Temperatur von 150°C Manteltemperatur bei einem Druck von kleiner 0,1 mbar.

Das Lösungsmittel und gegebenenfalls die Restmonomere werden im nachgeschalteten Kühlfallensystem ausgefroren und anschließend ausgetragen.

Die vorliegende Erfindung wird anhand nachfolgender Beispiele näher beschrieben. Sie ist aber nicht auf diese Beispiele beschränkt.

Herstellung eines Acrylat-Copolymers

Das Acrylat Co-Polymer wird mittels radikalischer Lösungsmittelpolymerisation bei Normaldruck unter Sieden bei 111°C im Zulaufverfahren hergestellt. Die Siedetemperatur der Polymerlösung steigt während der Reaktion auf ein Maximum von ungefähr 117°C. In das vorgelegte siedende Lösungsmittel werden gleichzeitig beginnend die Monomere in einem Zeitraum von 120 Minuten und der Initiator in einem Zeitraum von 240 Minuten dem Reaktor zugeführt.

Zusammensetzung und chemische sowie physikalische Eigenschaften des Acrylat-Copolymers

Tabelle 3 zeigt unterschiedliche Zusammensetzungen des Acrylat-Copolymers, welche die Basis zur Herstellung des erfindungsgemäßen Trenngels bilden. Als Komponenten für die Herstellung der unterschiedlichen Zusammensetzungen der Acrylat-Copolymere wurden als Lösungsmittel Toluol, als Monomere n-Butylacrylat (NBA) und 2-Ethylhexylacrylat (EHA) und als Initiator 1,1,3,3-Tetramethylbutylperoxy-2-ethylhexanoat (z.B. Trigonox® 421 von Nouryon (T421) oder Azobis(isobutyronitril) (AIBN)) verwendet.

In Tabelle 3 sind die Viskosität und die Dichte und die Restmengen der Monomere sowie des Lösungsmittels Toluol angeführt, wobei die Parameter mittels der oben angeführten Messmethoden bestimmt wurden.

Tabelle 3

| Nr. | NBA:EHA (Massenverhältnis) | Initiator | Viskosität 20°C [Pa·s] | Dichte 20°C [g/cm³] | Restmenge (Reinheit) | | |
|---|---|---|---|---|---|---|---|
| | | | | | Toluol [ppm] | NBA [ppm] | EHA [ppm] |
| 1 | 3:1 | AIBN | 109 | 1,026 (2 | 59 | <5 | 158 |
| 2 | 9:1 | T421 | 166 | 1,038 (1 | 19 | <5 | 60 |
| 3 | 9:1 | T421 | 67 | 1,038 (1 | 24 | <5 | <5 |
| 4 | 9:1 | T421 | 124 | 1,038 (1 | 6 | <5 | <5 |
| 5 | 3:1 | T421 | 64 | 1,027 (1 | 10 | <5 | 6 |
| 6 | 3:1 | T421 | 89 | 1,028 (1 | <5 | <5 | 24 |
| 7 | 4:1 | T421 | 100 | 1,032 (1 | 33 | <5 | 13 |
| 8 | 4:1 | T421 | 101 | 1,031 (2 | <5 | <5 | <5 |
| 9 | 4:1 | T421 | 100 | 1,031 (2 | 17 | <5 | 5 |
| (1 Messmethode: Gaspyknometer (2 Messmethode: Biegeschwinger Dichtemessgerät | | | | | | | |

Ergeben die Messresultate der GC-Messungen zur Restmenge von Toluol, NBA oder EHA Ergebnisse, die nicht nachweisbar sind bzw. kleiner 5 ppm sind, so sind sie in Tabelle 3 mit kleiner 5 aufgelistet.

Zusammensetzung und chemische sowie physikalische Eigenschaften des Trenngels

In Tabelle 4 sind die Zusammensetzung, die Viskosität und die Dichte von Trenngelen angeführt, die unter Verwendung der in Tabelle 3 beschriebenen Beispiele für Acrylat-Copolymere hergestellt wurden, angeführt, wobei die Parameter ebenfalls mittels der oben angeführten Messmethoden bestimmt wurden.

Tabelle 4

| N r. | Polymer | Kieselsäure hydrophil | [Ge w.-%] | Kieselsäure hydrophob | [Ge w.-%] | Silikonöl | Polyalkylenglykol | [Ge w.-%] | Titandioxid | Dichte 20°C [g/cm$^3$] | Viskosität bei 20° C [Pa· s] | TI |
|---|---|---|---|---|---|---|---|---|---|---|---|---|
| 1 | 2 | Aerosil 200 | 2,75 | - | - | Xiameter™ OFX-0193 | - | 0,15 | 0,012 5% | 1,055 [1] | N/A | N/ A |
| 2 | 3 | Aerosil 200 | 2,75 | Aerosil® R820 0 | 0,4 | Xiameter™ OFX-0193 | - | 0,15 | - | 1,054 [1] | N/A | N/ A |
| 3 | 3 | Aerosil 200 | 2,75 | Aerosil® R208 | 0,4 | Xiameter™ OFX-0193 | - | 0,15 | - | 1,053 [1] | N/A | N/ A |
| 4 | 4 | Aerosil 200 | 2,75 | - | - | Xiameter™ OFX-0190 | - | 0,15 | - | 1,054 [1] | N/A | N/ A |
| 5 | 4 | Aerosil 200 | 2,75 | Aerosil ®R97 4 | 0,4 | Xiameter™ OFX-0193 | - | 0,15 | - | 1,055 [1] | N/A | N/ A |
| 6 | 5 | Aerosil 200 | 2,75 | - | - | Xiameter™ OFX-0190 | - | 0,15 | 0,012 5% | 1,043 [1] | N/A | N/ A |
| 7 | 6 | Aerosil 200 | 2,65 | Aerosil® R974 | 1,0 | Xiameter™ OFX-0190 | - | 0,15 | 0,012 5% | 1,048 [1] | N/A | N/ A |
| 8 | 7 | Aerosil 200 | 2,75 | - | - | Xiameter™ OFX-0190 | - | 0,50 | 0,012 5% | 1,047 [1] | N/A | N/ A |
| 9 | 8 | Aerosil 200 | 2,75 | - | - | Xiameter™ OFX-0190 | - | 0,15 | 0,012 5% | 1,047 [2] | 230 | 1,4 |
| 10 | 9 | Aerosil 200 | 2,75 | - | - | - | PPG (Mn 624) | 0,15 | 0,012 5% | 1,047 [2] | 214 | 1,3 |
| 11 | 9 | Aerosil 200 | 2,75 | - | - | - | PPG (Mn 400) | 0,13 5 | 0,012 5% | 1,047 [2] | 211 | 1,3 |
| | | | | | | | PEG (Mn 400) | 0,01 5 | | | | |

[1] Messmethode: Gaspyknometer
[2] Messmethode: Biegeschwinger Dichtemessgerät
AEROSIL® Produktlinie von EVONIK Industries AG
XIAMETER™ Produktlinie von DOW Corning
Titandioxid KRONOS 1171

Alle oben angeführten Trenngele zeigten eine Restmenge von 10 ppm bis 50 ppm Toluol und ≤ 20 ppm n-Butylacrylat und ≤ 80 ppm 2-Ethylhexylacrylat. Die Messwerte der Restmengen von Lösungsmittel und Monomer der für die Herstellung des Trenngels verwendeten Acrylat-Copolymere gelten auch für die Trenngele, weil im Mischprozess der Komponenten zur Herstellung des Trenngels kein weiterer Eintrag von Monomeren oder Lösungsmittel erfolgt.

In einer bevorzugten Ausführungsvariante weist das Trenngel eine Zusammensetzung aus 97,1 Gew.-% aufgereinigtes Acrylat-Copolymer, 2,75 Gew.-% Kieselsäure, 0,15 Gew.-% Silikonöl und 0,0125 Gew-% Titandioxid auf.

In einer weiteren Ausführungsvariante weist das Trenngel eine Zusammensetzung aus 97,1 Gew.-% aufgereinigtes Acrylat-Copolymer, 2,75 Gew.-% Kieselsäure, 0,15 Gew.-% PEG und/oder PPG und 0,0125 Gew.-% Titandioxid auf.

Eine mögliche Ausführungsvariante des Trenngels umfasst ein Acrylat-Copolymer, Kieselsäure und ein Silikonöl und zumindest ein Polyalkylenglycol. Diese Ausführungsform besteht vorzugsweise aus 97,1 Gew.-% aufgereinigtes Acrylat-Copolymer, 2,75 Gew.-% Kieselsäure und einer Gesamtmenge von 0,15 Gew.-% Silikonöl, PEG und PPG sowie 0,0125 Gew.-% Titandioxid.

In einer Studie wurden einerseits Blutentnahmeröhrchen zur Gewinnung von Serum und andererseits Blutentnahmeröhrchen zur Gewinnung von Plasma mit dem erfindungsgemäßen Trenngel (A, D) und mit einem etablierten, am Markt zugelassen Trenngel (H, J) verglichen. Hierzu wurde 20 Probanden mit unterschiedlichen Blutentnahmeröhrchen Blut entnommen und dieses untersucht. Die Mittelwerte der bestimmten Analyten in den Blutentnahmeröhrchen mit dem erfindungsgemäßen Trenngel wurden mit den Mittelwerten der bestimmten Analyten in den Blutentnahmeröhrchen mit dem am Markt zugelassen Trenngel derselben 20 Probanden verglichen.

Nachfolgende Tabelle 5 zeigt die Messergebnisse von Blutentnahmeröhrchen zur Gewinnung von Serum.

Tabelle 5

| Parameter | Gruppe | A Mittelwert | H Mittelwert | Kriterium % | Äquivalenz |
|---|---|---|---|---|---|
| Natrium | Elektrolyte | 138,85 | 138,70 | 3,00 | Ja |
| Kalium | Elektrolyte | 4,09 | 4,07 | 4,50 | Ja |
| Chlorid | Elektrolyte | 104,25 | 104,25 | 4,50 | Ja |
| Gesamtprotein | Proteine | 68,12 | 67,18 | 6,00 | Ja |
| Albumin | Proteine | 43,51 | 43,56 | 8,00 | Ja |
| Bilirubin | Leberfunktion | 0,68 | 0,68 | 20,00 | Ja |
| Alanin-Aminotransferase | Leberfunktion | 25,60 | 25,60 | 11,50 | Ja |
| Glukose | Allgemeine Tests | 95,40 | 95,45 | 8,00 | Ja |
| Calcium | Allgemeine Tests | 2,39 | 2,39 | 6,00 | Ja |
| Kreatinin | Nierenfunktion | 0,84 | 0,84 | 11,00 | Ja |

In Tabelle 6 werden die Ergebnisse der Analyten von Blutentnahmeröhrchen zur Gewinnung von Plasma gezeigt.

Tabelle 6

| Parameter | Gruppe | D Mittelwert | J Mittelwert | Kriterium % | Äquivalenz |
|---|---|---|---|---|---|
| Natrium | Elektrolyte | 138,75 | 138,55 | 3,00 | Ja |
| Kalium | Elektrolyte | 3,79 | 3,80 | 4,50 | Ja |
| Chlorid | Elektrolyte | 104,40 | 104,45 | 4,50 | Ja |
| Gesamtprotein | Proteine | 69,60 | 69,36 | 6,00 | Ja |
| Albumin | Proteine | 43,16 | 43,13 | 8,00 | Ja |
| Bilirubin | Leberfunktion | 0,68 | 0,68 | 20,00 | Ja |
| Alanin-Aminotransferase | Leberfunktion | 25,55 | 25,00 | 11,50 | Ja |
| Glukose | Allgemeine Tests | 96,05 | 97,20 | 8,00 | Ja |
| Calcium | Allgemeine Tests | 2,37 | 2,37 | 6,00 | Ja |

(fortgesetzt)

| Parameter | Gruppe | D Mittelwert | J Mittelwert | Kriterium % | Äquivalenz |
|---|---|---|---|---|---|
| Kreatinin | Nierenfunktion | 0,84 | 0,83 | 11,00 | Ja |

Basierend auf dem umfassenden metabolischen Panel wurden ein oder mehrere Parameter je Gruppe (Allgemeine Tests, Elektrolyte, Proteine, Leberfunktion, Nierenfunktion) für die Studie ausgewählt.

Die ausgewählten Parameter überprüfen im ersten Schritt den Gesundheitszustand und liefern ein Gesamtbild des chemischen Gleichgewichts im Körper als auch des Metabolismus, sowohl in der Routineuntersuchung als auch der stationären oder ambulanten Untersuchung.

In der Spalte Kriterium in % werden die in der klinischen Chemie gängigen Akzeptanzkriterien (zum Beispiel RILIBÄK, CLIR) angeführt. Äquivalenz zwischen den Blutentnahmeröhrchen mit dem erfindungsgemäßen Trenngel und dem aus dem Stand der Technik bekannten Trenngel wurde dann festgestellt, wenn der prozentuale Unterschied der beiden Mittelwerte unter dem Akzeptanzkriterium liegt.

In der Studie konnten für die angegeben Parameter keine klinisch signifikanten Unterschiede zwischen Blutentnahmeröhrchen mit dem erfindungsgemäßen Trenngel und dem am Markt zugelassenen etablierten Trenngel festgestellt werden.

Die Ausführungsbeispiele zeigen mögliche Ausführungsvarianten, wobei an dieser Stelle bemerkt sei, dass die Erfindung nicht auf die speziell dargestellten Ausführungsvarianten derselben eingeschränkt ist, sondern vielmehr auch diverse Kombinationen der einzelnen Ausführungsvarianten untereinander möglich sind und diese Variationsmöglichkeit aufgrund der Lehre zum technischen Handeln durch gegenständliche Erfindung im Können des auf diesem technischen Gebiet tätigen Fachmannes liegt.

Der Schutzbereich ist durch die Ansprüche bestimmt. Die Beschreibung und die Zeichnungen sind jedoch zur Auslegung der Ansprüche heranzuziehen. Einzelmerkmale oder Merkmalskombinationen aus den gezeigten und beschriebenen unterschiedlichen Ausführungsbeispielen können für sich eigenständige erfinderische Lösungen darstellen. Die den eigenständigen erfinderischen Lösungen zugrundeliegende Aufgabe kann der Beschreibung entnommen werden.

Sämtliche Angaben zu Wertebereichen in gegenständlicher Beschreibung sind so zu verstehen, dass diese beliebige und alle Teilbereiche daraus mitumfassen, z.B. ist die Angabe 1 bis 10 so zu verstehen, dass sämtliche Teilbereiche, ausgehend von der unteren Grenze 1 und der oberen Grenze 10 mit umfasst sind, d.h. sämtliche Teilbereiche beginnen mit einer unteren Grenze von 1 oder größer und enden bei einer oberen Grenze von 10 oder weniger, z.B. 1 bis 1,7, oder 3,2 bis 8,1, oder 5,5 bis 10.

Der Ordnung halber sei abschließend darauf hingewiesen, dass zum besseren Verständnis des Aufbaus Elemente teilweise unmaßstäblich und/oder vergrößert und/oder verkleinert dargestellt wurden.

**Patentansprüche**

1. Trenngel für Blutentnahmeröhrchen zur Separation von Blutserum oder -plasma von Blutzellen umfassend ein Acrylat-Copolymer, Kieselsäure und Silikonöl und/oder zumindest ein Polyalkylenglykol, **dadurch gekennzeichnet, dass** im Trenngel ≤ 1000 ppm, vorzugsweise ≤ 300 ppm, Lösungsmittel enthalten sind.

2. Trenngel nach Anspruch 1, **dadurch gekennzeichnet, dass** zur Polymerisation des Acrylat-Copolymers zur Herstellung einer Polymerkomponente zumindest zwei Monomere, insbesondere n-Butylacrylat und 2-Ethylhexylacrylat, ein Lösungsmittel und ein Initiator eingesetzt sind.

3. Trenngel nach Anspruch 1 oder 2, **dadurch gekennzeichnet, dass** das Monomerverhältnis n-Butylacrylat zu 2-Ethylhexylacrylat 2:1 bis 9:1, insbesondere 3:1 bis 5:1, vorzugsweise 4:1, zur Herstellung der Polymerkomponente beträgt.

4. Trenngel nach zumindest einem der Ansprüche 1 bis 3, **dadurch gekennzeichnet, dass** im Acrylat-Copolymer ≤ 1000 ppm, vorzugsweise ≤ 300 ppm, Lösungsmittel enthalten sind.

5. Trenngel nach zumindest einem der Ansprüche 1 bis 4, **dadurch gekennzeichnet, dass** das Acrylat-Copolymer einen Restgehalt von ≤ 50 ppm, insbesondere ≤ 20 ppm, n-Butylacrylatmonomere aufweist.

6. Trenngel nach zumindest einem der Ansprüche 1 bis 5, **dadurch gekennzeichnet, dass** das Acrylat-Copolymer einen Restgehalt von ≤ 200 ppm, vorzugsweise ≤ 100 ppm, insbesondere ≤ 80 ppm, 2-Ethylhexylacrylatmonomere aufweist.

7. Verfahren zur Herstellung eines Trenngels nach einem der Ansprüche 1 bis 6 für Blutentnahmeröhrchen zur Separation von Blutserum oder -plasma umfassend ein Acrylat-Copolymer, Kieselsäure und Silikonöl und/oder zumindest ein Polyalkylenglykol, **dadurch gekennzeichnet, dass** das Acrylat-Copolymer, Kieselsäure und Silikonöl und/oder zumindest ein Polyalkylenglykol und gegebenenfalls Titandioxid gemischt werden.

8. Verfahren zur Herstellung eines Trenngels nach Anspruch 7, **dadurch gekennzeichnet, dass** zur Herstellung einer Polymerkomponente des Acrylat-Copolymers zumindest n-Butylacrylat in einer Menge von 30 Gew.-% bis 50 Gew.-%, vorzugsweise 35 Gew.-% bis 45 Gew.-%, insbesondere 38 Gew.-% bis 42 Gew.-%, und 2-Ethylhexylacrylat in einer Menge von 2 Gew.-% bis 20 Gew.-%, vorzugsweise 5 Gew.-% bis 15 Gew.-%, insbesondere 8 Gew.-% bis 12 Gew.-% verwendet werden.

9. Verfahren zur Herstellung eines Trenngels nach Anspruch 7 oder 8, **dadurch gekennzeichnet, dass** als Lösungsmittel, ein aromatisches Lösungsmittel, vorzugsweise Toluol und/oder Xylol, zur Herstellung der Polymerkomponente, vorzugsweise in einer Menge von 30 Gew.-% bis 70 Gew.-%, vorzugsweise 40 Gew.-% bis 60 Gew.-%, insbesondere 45 Gew.-% bis 55 Gew.-%, verwendet wird.

10. Verfahren zur Herstellung eines Trenngels nach zumindest einem der Ansprüche 7 bis 9, **dadurch gekennzeichnet, dass** als Initiator zur Polymerisation der zumindest zwei Monomere ein organisches Peroxid, insbesondere 1,1,3,3-Tetramethylbutylperoxy-2-ethylhexanoat, zur Herstellung der Polymerkomponente, vorzugsweise in einer Menge von 0,05 Gew.-% bis 5 Gew.-%, vorzugsweise 0,1 Gew.-% bis 1 Gew.-%, insbesondere 0,3 Gew.-% bis 0,6 Gew.-%, verwendet wird.

11. Blutentnahmeröhrchen zur Separation von Blutserum oder -plasma von Blutzellen mit einem Trenngel nach einem der Ansprüche 1 bis 6.

12. Verfahren zur Herstellung eines Acrylat-Copolymers für ein Trenngel für Blutentnahmeröhrchen durch radikalische Lösungsmittelpolymerisation im Zulaufverfahren aus einer Polymerkomponente, **dadurch gekennzeichnet, dass** zur Aufreinigung des Acrylat-Copolymers zumindest ein Lösungsmittel durch mehrstufige Destillation abgetrennt wird, sodass ein Lösungsmittelrestgehalt von ≤ 1000 ppm, insbesondere ≤ 300 ppm, erreicht wird.

13. Verfahren nach Anspruch 12, **dadurch gekennzeichnet, dass** die mehrstufige Destillation als Dünnschichtdestillation durchgeführt wird und dadurch Restmonomere aus der Polymerkomponente des Acrylat-Copolymers abgetrennt werden, sodass ≤ 50 ppm, insbesondere ≤ 20 ppm, eines ersten Monomers, insbesondere n-Butylacrylat, und ≤ 200 ppm, vorzugsweise ≤ 100 ppm, insbesondere ≤ 80 ppm, eines zweiten Monomers, insbesondere 2-Ethylhexylacrylat, erreicht werden.

14. Verfahren nach zumindest einem der Ansprüche 12 oder 13, **dadurch gekennzeichnet, dass** in einer anschließenden Kurzwegverdampfungsstufe das Lösungsmittel und gegebenenfalls die Restmonomere auf den Endgehalt abdestilliert werden.

**Fig.1**

Europäisches
Patentamt

European
Patent Office

Office européen
des brevets

# EUROPÄISCHER RECHERCHENBERICHT

Nummer der Anmeldung

EP 25 17 2981

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | EP 3 279 654 A1 (SEKISUI MEDICAL CO LTD [JP]) 7. Februar 2018 (2018-02-07) | 1,2,4-7, 11 | INV. B01J20/26 |
| Y | * Vergleichsbeispiel 3; Beispiele 1-4, 6; Tabellen 1, 2 * ----- | 10 | B01J20/10 B01J20/06 B01J20/28 |
| X | EP 4 317 969 A1 (SEKISUI MEDICAL CO LTD [JP]) 7. Februar 2024 (2024-02-07) * Vergleichsbeispiel 2; Tabelle 1 * ----- | 1,2,4-7, 11 | G01N33/49 G01N33/50 A61B5/15 |
| X,D | US 5 438 000 A (LEGARIO RONALD [JP] ET AL) 1. August 1995 (1995-08-01) * Beispiele 1-16; Tabellen 2, 3 * ----- | 1-9,11 | |
| X | WO 2007/029525 A1 (TOAGOSEI CO LTD [JP]; SEKISUI CHEMICAL CO LTD [JP] ET AL.) 15. März 2007 (2007-03-15) | 12-14 | |
| Y | * Absatz [0021] * * Absätze [0048], [0049] * ----- | 10 | |

|  |
|---|
| RECHERCHIERTE SACHGEBIETE (IPC) |
| G01N B01J |

Der vorliegende Recherchenbericht wurde für alle Patentansprüche erstellt

| Recherchenort | Abschlußdatum der Recherche | Prüfer |
|---|---|---|
| Den Haag | 2. Dezember 2025 | Klemps, Christian |

EPO FORM 1503 03.82 (P04C03)

## ANHANG ZUM EUROPÄISCHEN RECHERCHENBERICHT
## ÜBER DIE EUROPÄISCHE PATENTANMELDUNG NR.

EP 25 17 2981

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

02-12-2025

| Im Recherchenbericht angeführtes Patentdokument | Datum der Veröffentlichung | Mitglied(er) der Patentfamilie | Datum der Veröffentlichung |
|---|---|---|---|
| EP 3279654 A1 | 07-02-2018 | CN 107209169 A | 26-09-2017 |
| | | EP 3279654 A1 | 07-02-2018 |
| | | JP 6172832 B2 | 02-08-2017 |
| | | JP WO2016159236 A1 | 07-09-2017 |
| | | KR 20170132135 A | 01-12-2017 |
| | | US 2018092806 A1 | 05-04-2018 |
| | | WO 2016159236 A1 | 06-10-2016 |
| EP 4317969 A1 | 07-02-2024 | CN 117157523 A | 01-12-2023 |
| | | EP 4317969 A1 | 07-02-2024 |
| | | JP 7309108 B2 | 18-07-2023 |
| | | JP WO2022202873 A1 | 29-09-2022 |
| | | KR 20230159830 A | 22-11-2023 |
| | | US 2024172974 A1 | 30-05-2024 |
| | | WO 2022202873 A1 | 29-09-2022 |
| US 5438000 A | 01-08-1995 | CA 2102993 A1 | 13-05-1994 |
| | | EP 0597690 A2 | 18-05-1994 |
| | | JP 3260219 B2 | 25-02-2002 |
| | | JP H06201682 A | 22-07-1994 |
| | | US 5438000 A | 01-08-1995 |
| WO 2007029525 A1 | 15-03-2007 | JP 4510893 B2 | 28-07-2010 |
| | | JP WO2007029525 A1 | 19-03-2009 |
| | | WO 2007029525 A1 | 15-03-2007 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5438000 A
- EP 3734273 A1